# EUROPÄISCHE PATENTSCHRIFT

(11) **EP 1 713 802 B1**
(45) Veröffentlichungstag und Bekanntmachung des Hinweises auf die Patenterteilung: **25.07.2007**
(21) Anmeldenummer: 05700907.8
(22) Anmeldetag: 14.01.2005
(51) Int. Cl.: C07D 471/04, C07D 471/16, C07D 487/16, C07D 513/04, C09B 57/00

(54) **MESOIONISCHE PIGMENTE AUF BASIS VON 2,4-DIOXOPYRIMIDINEN**
2,4-DIOXOPYRIMIDINE-BASED MESOIONIC PIGMENTS
PIGMENTS MESOIONIQUES A BASE DE 2,4-DIOXOPYRIMIDINES

(30) Priorität: 27.01.2004 DE 102004003888
(43) Veröffentlichungstag der Anmeldung: 25.10.2006
(73) Patentinhaber: Clariant Produkte (Deutschland) GmbH, 65929 Frankfurt am Main (DE)
(72) Erfinder: METZ, Thomas, 64646 Heppenheim (DE); PLÜG, Carsten, 64342 Seeheim-Jugenheim/Ober-Beerbach (DE)
(74) Vertreter: Hütter, Klaus
(86) Internationale Anmeldenummer: PCT/EP2005/000305
(87) Internationale Veröffentlichungsnummer: WO 2005/070928

(56) Entgegenhaltungen:
- US-A- 4 525 591

## Beschreibung

Die vorliegende Erfindung betrifft neue, mesoionische Verbindungen, ein Verfahren zu ihrer Herstellung und ihre Verwendung als Pigmente.

In der Literatur [W. Friedrichsen, T. Kappe, A. Böttcher, Heterocycles 1982, 19, 1083-1148; C. Wentrup et al., J.Chem.Soc, Perkin Trans 2 2000, 2096-2108] sind mesoionische Verbindungen der Formel (I) 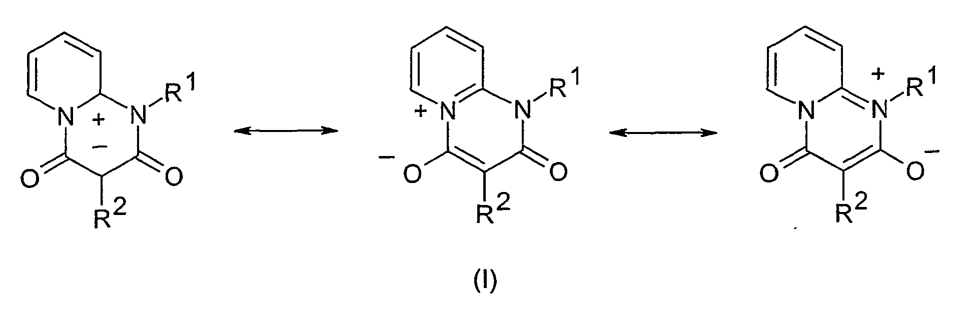 beschrieben, wobei die Radikale R¹ für Methyl oder Phenyl und R² für Methyl oder Wasserstoff stehen. Im Folgenden wird für Mesoionen nur die zuerst gezeigte Darstellungsweise verwendet.
Diese Verbindungen zeigen hohe Schmelzpunkte und sind als gelbe Kristalle beschrieben. Bei Ihrer Herstellung fiel auf, dass sie Festkörperfluoreszenz zeigen, aber als Pigmente aufgrund ihrer geringen Farbstärke nicht geeignet sind.

Festkörperfluoreszierende Pigmente sind ausgesprochen selten. Kommerziell erhältliche Farbmittel wie Pigment Gelb 101 und die so genannten Fluoreszenzpigmente, die auf Inkorporation von Farbstoffen in Polymere beruhen, befriedigen den Bedarf nicht, da die Lichtbeständigkeit dieser Pigmente für viele Anwendungsgebiete nicht ausreicht.

Es bestand die Aufgabe, leuchtend bunte und vorzugsweise fluoreszierende Pigmente bereitzustellen, die die hohen Echtheitseigenschaften von Pigmenten, wie Temperaturbeständigkeit, Licht- und Lösemittelechtheit sowie Migrationsbeständigkeit, mit der Brillanz von Fluoreszenzfarbstoffen verbinden.

Überraschenderweise wurde gefunden, dass eine Dimerisierung von Verbindungen der Formel (I) über eine Phenylenbrücke die Farbstärke deutlich erhöht und zudem Materialien mit Pigmentcharakter erhalten werden.

Gegenstand der vorliegenden Erfindung sind dimere Verbindungen der Formel (II) 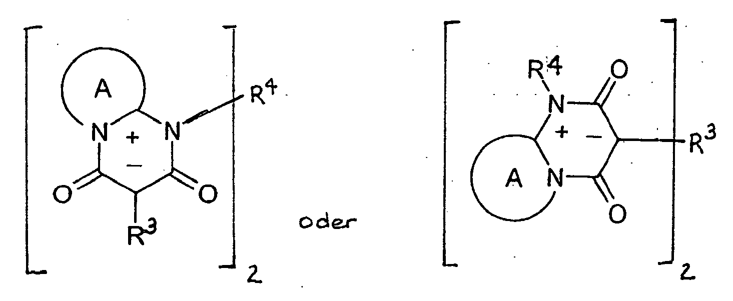 wobei
der Ring A ein fünf- oder sechsgliedriger heteroaromatischer Ring der Struktur A1 bis A7 ist 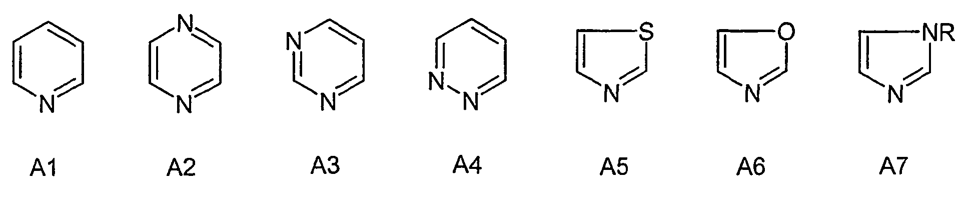 wobei die Ringe A1 bis A7 unsubstituiert, mit C₁-C₄-Alkyl oder Phenyl substituiert und/oder mit einem Benzolring anelliert sind,
einer der Reste R³ oder R⁴ ein unsubstituiertes oder ein mit Alkyl, Alkoxy und/oder Halogen substituiertes Phenylenradikal,
der andere der Reste R³ oder R⁴ C₁-C₄-Alkyl, C₅-C₆-Cycloalkyl, ein unsubstituiertes oder ein mit Alkyl, Alkoxy, Nitro, Phenyl, Alkoxycarbonyl, Dialkylamino, Dialkylaminocarbonyl, Alkylaminocarbonyl, Aminocarbonyl und/oder Halogen substituiertes Phenyl, Benzyl, Benzanilid, C₅-C₆-Cycloalkyl oder Naphthyl; oder wobei die Gruppe NR⁴ zusammen mit dem Ring A einen 5- oder 6-gliedrigen Heterocyclus, der noch mit einem Benzolring anelliert sein kann, bildet, und R³ ein unsubstituiertes oder ein mit Alkyl, Alkoxy und/oder Halogen substituiertes Phenylenradikal ist; und
R ein C₁-C₄-Alkyl oder Phenyl bedeutet.

Bevorzugte Verbindungen im Sinne der vorliegenden Erfindung sind Verbindungen der allgemeinen Formeln (IIa) und (IIb) 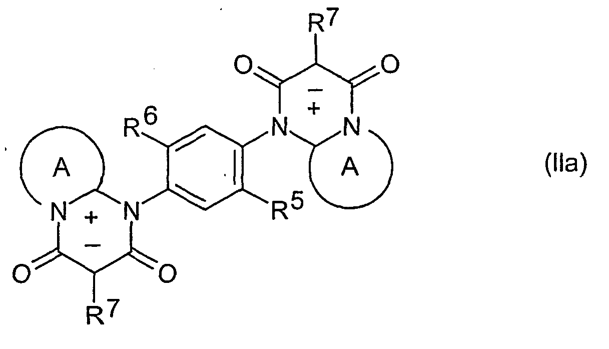 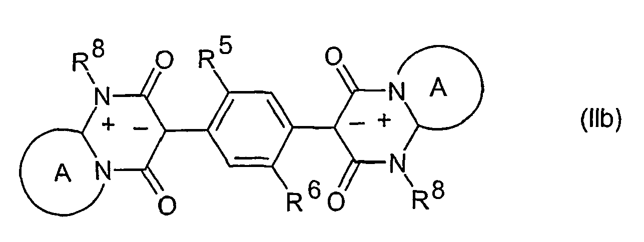 wobei A wie zuvor definiert ist,
R⁵ und R⁶ unabhängig voneinander für Wasserstoff, C₁-C₄-Alkyl, C₁-C₄-Alkoxy oder Halogen, vorzugsweise für Wasserstoff, Methyl oder Chlor, stehen;
R⁷ und R⁸ für C₁-C₄-Alkyl, C₅-C₆-Cycloalkyl, ein unsubstituiertes oder ein mit 1, 2, 3 oder 4 Resten aus der Gruppe C₁-C₄-Alkyl, C₁-C₄-Alkoxy, Nitro, Phenyl, C₁-C₄-Alkoxycarbonyl, Di(C₁-C₃-Alkyl)amino, Di(C₁-C₃-Alkyl)-aminocarbonyl, (C₁-C₃-Alkyl)-aminocarbonyl, Aminocarbonyl und/oder Chlor substituiertes Phenyl, Benzyl, Benzanilid oder Naphthyl stehen;
oder wobei die Gruppe NR⁸ zusammen mit dem Ring A einen 5- oder 6-gliedrigen Heterocyclus, der noch mit einem Benzolring anelliert sein kann, wie z.B. Pyrrol oder ein Benzopyrrol, bildet.

Das zuvor in der Definition für R³, R⁴, R⁷ und R⁸ genannte Phenylradikal ist bevorzugt ausgewählt aus der Gruppe 1-, 2-, 3-Methyl-, Ethyl-, Methoxy-, Ethoxy-, Diethylamino-, Chlorphenyl, 2,5-Dichlor-, 3-Chlor-4-methyl-, 3-Chlor-4-methoxy- und 4-Nitrophenyl.

Besonders bevorzugt sind Verbindungen der Formel (V) 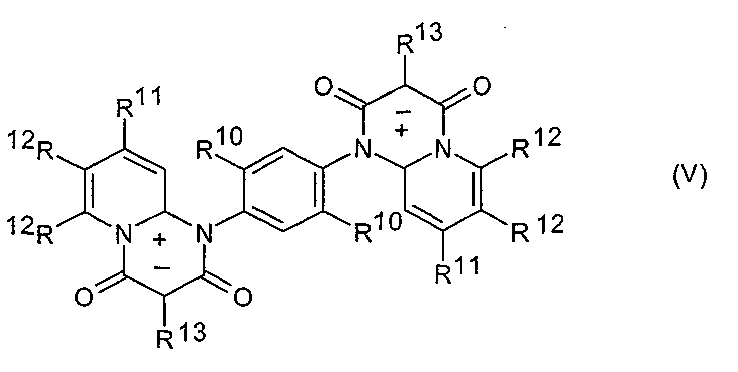 worin
- R¹⁰: Wasserstoff, Methyl oder Chlor,
- R¹¹: Wasserstoff oder Methyl,
- R¹²: Wasserstoff oder zwei benachbarte Reste R¹² gemeinsam den zweiwertigen Rest C₄H₄, und
- R¹³: Methyl oder Phenyl bedeuten.

Je nach Verknüpfung der mesoionischen Verbindungen gemäß Formel (IIa) oder (IIb) eröffnet sich für die Pigmente ein breites Farbspektrum, das einen Zugang zu gelben, roten und grünen Pigmenten ermöglicht.

Die erfindungsgemäßen Verbindungen der Formel (II) können aus N-substituierten Ringen vom Typ A mit Amidinstruktur (III) und substituierten Malonylchloriden (IV) nach dem folgenden Schema hergestellt werden. 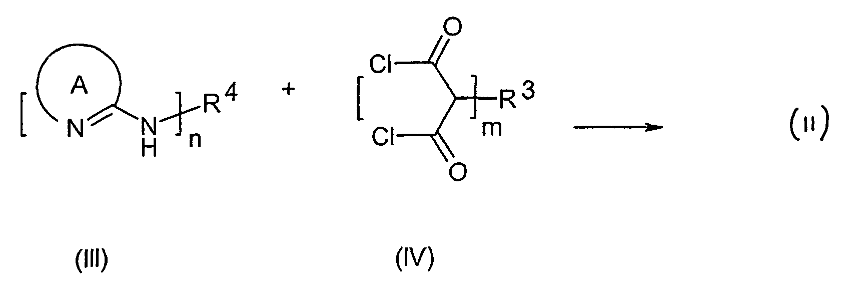 wobei entweder m = 2 und n = 1, oder m = 1 und n = 2 bedeuten.

Gegenstand der Erfindung ist auch ein Verfahren zur Herstellung von Verbindungen der Formel (II), dadurch gekennzeichnet, dass man entweder
(a) ein Equivalent der Verbindung der Formel (III) mit n gleich 2 mit etwa zwei Equivalenten der Verbindung der Formel (IV) mit m gleich 1; oder
(b) ein Equivalent der Verbindung der Formel (IV) mit m gleich 2 mit etwa zwei Equivalenten der Verbindung der Formel (III) mit n gleich 1,
kondensiert.

Im Fall von (a) werden in das Reaktionsgemisch vorteilhaft auf ein Mol-Equivalent der Verbindung (III) 2 bis 5 Mol-Equivalente, vorzugsweise 3,5 bis 4 Mol-Equivalente, der Verbindung (IV) eingesetzt.
Im Fall von (b) werden in das Reaktionsgemisch vorteilhaft auf ein Mol-Equivalent der Verbindung (IV) 2 bis 3 Mol-Equivalente, vorzugsweise 2 bis 2,5 Mol-Equivalente, der Verbindung (III) eingesetzt.

Die Verbindungen der Formeln (III) und (IV) sind literaturbekannt oder analog zu bekannten Verfahren zugänglich [T. Hisano, T. Matsuoka, K. Tsutsumi, K. Muraoka, M. Ichikawa, Chem. Pharm. Bull., 1981, 29 (12), 3706-3712; P. Laackmann, W. Friedrichsen, Tetrahedron, 1996, 52, 5475-5486.].

Als besonders vorteilhaft hat es sich erwiesen, die Kondensation in Gegenwart einer Base, bevorzugt von 1 bis 5 Equivalenten, insbesondere 2 bis 5 Equivalenten, einer Base, bezogen auf das Amidin (III), durchzuführen. Als Base kommt z.B. Triethylamin, Pyridin, Picolin, N-Methylimidazol oder Alkalicarbonat in Betracht.
Es ist jedoch auch möglich, die Kondensation ohne Zugabe einer Base durchzuführen wobei jedoch durch unvollständige Kondensation die Ausbeute an Verbindung (11) kleiner werden kann.
Die Kondensation wird zweckmäßigerweise in einem aprotischen Lösungsmittel, bevorzugt Methylenchlorid, Chloroform, N-Methylpyrrolidon, Dimethylformamid, o-Dichlorbenzol, Chlorbenzol, Ethylacetat, Butylacetat, Aceton, Methylethylketon, Methyl-i-butylketon, Methyl-t-butylether, Tetrahydrofuran, Dioxan oder Propylenglykoldimethyl-ether, bei Temperaturen von -10 bis +100°C, bevorzugt 50 bis 80°C, durchgeführt.
Die erfindungsgemäßen Verbindungen werden abfiltriert, von Reaktionsnebenprodukten durch Nachwaschen befreit, zum Abtrennen der Salze mit Wasser ausgerührt, abfiltriert und gegebenenfalls getrocknet.

Zur Verbesserung der pigmentären Eigenschaften können die erfindungsgemäßen Verbindungen noch einer Feinverteilung, z.B. einer Mahlung, und/oder einer Wärmebehandlung in wässrigem, wässrig-organischem oder organischem Medium bei Temperaturen zwischen 40°C und 200°C, gegebenenfalls unter Druck unterworfen werden. Anschließend können die erhaltenen Pigmentsuspensionen auf die übliche Weise filtriert, der Presskuchen mit Wasser salzfrei gewaschen, getrocknet und gemahlen werden.

Die erfindungsgemäßen Verbindungen lassen sich zum Pigmentieren von hochmolekularen organischen Materialien natürlicher oder synthetischer Herkunft einsetzen, beispielsweise von Kunststoffen, Harzen, Lacken, Anstrichfarben, elektrophotographischen Tonern und Entwicklern, Elektretmaterialien, Farbfilter sowie von Tinten, Druckfarben und Saatgut.

Hochmolekulare organische Materialien, die mit den erfindungsgemäßen Verbindungen pigmentiert werden können, sind beispielsweise Celluloseverbindungen, wie beispielsweise Celluloseether und -ester, wie Ethylcellulose, Nitrocellulose, Celluloseacetate oder Cellulosebutyrate, natürliche Bindemittel, wie beispielsweise Fettsäuren, fette Öle, Harze und deren Umwandlungsprodukte, oder Kunstharze, wie Polykondensate, Polyaddukte, Polymerisate und Copolymerisate, wie beispielsweise Aminoplaste, insbesondere Harnstoff- und Melaminformaldehydharze, Alkydharze, Acrylharze, Phenoplaste und Phenolharze, wie Novolake oder Resole, Harnstoffharze, Polyvinyle, wie Polyvinylalkohole, Polyvinylacetale, Polyvinylacetate oder Polyvinylether, Polycarbonate, Polyolefine, wie Polystyrol, Polyvinylchlorid, Polyethylen oder Polypropylen, Poly(meth)acrylate und deren Copolymerisate, wie Polyacrylsäureester oder Polyacrylnitrile, Polyamide, Polyester, Polyurethane, Cumaron-Inden- und Kohlenwasserstoffharze, Epoxidharze, ungesättigte Kunstharze (Polyester, Acrylate) mit den unterschiedlichen Härtemechanismen, Wachse, Aldehyd- und Ketonharze, Gummi, Kautschuk und seine Derivate und Latices, Casein, Silikone und Silikonharze; einzeln oder in Mischungen.
Dabei spielt es keine Rolle, ob die erwähnten hochmolekularen organischen Verbindungen als plastische Massen, Schmelzen oder in Form von Spinnlösungen, Dispersionen, Lacken, Anstrichstoffen oder Druckfarben vorliegen. Je nach Verwendungszweck erweist es sich als vorteilhaft, die erfindungsgemäßen Verbindungen als Blend oder in Form von Präparationen oder Dispersionen zu benutzen. Bezogen auf das zu pigmentierende, hochmolekulare organische Material setzt man die erfindungsgemäßen Verbindungen in einer Menge von 0,05 bis 30 Gew.-%, vorzugsweise 0,1 bis 15 Gew.-%, ein.
Es ist in manchen Fällen auch möglich, anstelle einer gemahlenen und/oder gefinishten erfindungsgemäßen Verbindung ein entsprechendes Crude mit einer BET-Oberfläche von größer als 2 m²/g, bevorzugt größer als 5 m²/g, einzusetzen. Dieser Crude kann zur Herstellung von Farbkonzentraten in flüssiger oder fester Form in Konzentrationen von 5 bis 99 Gew.-%, allein oder gegebenenfalls in Mischung mit anderen Crudes oder Fertigpigmenten, verwendet werden.

Die erfindungsgemäßen Verbindungen sind auch geeignet als Farbmittel in elektrophotographischen Tonern und Entwicklern, wie beispielsweise Ein- oder Zweikomponentenpulvertonern (auch Ein- oder Zweikomponenten-Entwickler genannt), Magnettoner, Flüssigtoner, Latextoner, Polymerisationstoner sowie Spezialtoner.
Typische Tonerbindemittel sind Polymerisations-, Polyadditions- und Polykondensationsharze, wie Styrol-, Styrolacrylat-, Styrolbutadien-, Acrylat-, Polyester-, Phenol-Epoxidharze, Polysulfone, Polyurethane, einzeln oder in Kombination, sowie Polyethylen und Polypropylen, die noch weitere Inhaltsstoffe, wie Ladungssteuermittel, Wachse oder Fließhilfsmittel, enthalten können oder im nachhinein mit diesen Zusätzen modifiziert werden.

Des weiteren sind die erfindungsgemäßen Verbindungen geeignet als Farbmittel in Pulver und Pulverlacken, insbesondere in triboelektrisch oder elektrokinetisch versprühbaren Pulverlacken, die zur Oheflächenbeschichtung von Gegenständen aus beispielsweise Metall, Holz, Kunststoff, Glas, Keramik, Beton, Textilmaterial, Papier oder Kautschuk zur Anwendung kommen.
Als Pulverlackharze werden typischerweise Epoxidharze, carboxyl- und hydroxylgruppenhaltige Polyesterharze, Polyurethan- und Acrylharze zusammen mit üblichen Härtern eingesetzt. Auch Kombinationen von Harzen finden Verwendung. So werden beispielsweise häufig Epoxidharze in Kombination mit carboxyl- und hydroxylgruppenhaltigen Polyesterharzen eingesetzt. Typische Härterkomponenten (in Abhängigkeit vom Harzsystem) sind beispielsweise Säureanhydride, Imidazole sowie Dicyandiamid und deren Abkömmlinge, verkappte Isocyanate, Bisacylurethane, Phenol- und Melaminharze, Triglycidylisocyanurate, Oxazoline und Dicarbonsäuren.

Gegenstand der Erfindung ist weiterhin die Verwendung der beschriebenen Farbmittelpräparation als Farbmittel für Drucktinten, insbesondere für Ink-Jet-Tinten.

Unter Ink-Jet-Tinten versteht man sowohl Tinten auf wässriger (einschließlich Mikroemulsionstinten) und nicht-wässriger ("solvent-based") Basis, UV-härtbare Tinten sowie solche Tinten, die nach dem Hot-Melt-Verfahren arbeiten.

Ink-Jet-Tinten auf Lösungsmittelbasis enthalten im Wesentlichen 0,5 bis 30 Gew.-%, vorzugsweise 1 bis 15 Gew.-%, einer oder mehrerer der erfindungsgemäßen Verbindungen, 70 bis 95 Gew.-% eines organischen Lösungsmittels oder Lösungsmittelgemisches und/oder einer hydrotropen Verbindung. Gegebenenfalls können die lösemittelbasierenden Ink-Jet-Tinten Trägermaterialien und Bindemittel enthalten, die im "Solvens" löslich sind, wie z.B. Polyolefine, Natur- und Synthesekautschuk, Polyvinylchlorid, Vinylchlorid/Vinylacetat-Copolymerisate, Polyvinylbutyrale, Wachs/Latex-Systeme oder Kombinationen dieser Verbindungen.
Gegebenenfalls können die lösungsmittelbasierenden Ink-Jet-Tinten noch weitere Zusatzstoffe enthalten, wie z.B. Netzmittel, Entgaser/Entschäumer, Konservierungsmittel und Antioxidantien.
Mikroemulsionstinten basieren auf organischen Lösemitteln, Wasser und gegebenenfalls eine zusätzlichen Substanz, die als Grenzflächenvermittler wirkt (Tensid). Mikroemulsionstinten enthalten 0,5 bis 30 Gew.-%, vorzugsweise 1 bis 15 Gew.-%, einer oder mehrerer der erfindungsgemäßen Verbindungen, 0,5 bis 95 Gew.-% Wasser und 0,5 bis 95 Gew.-% organische Lösungsmittel und/oder Grenzflächenvermittler.

UV-härtbare Tinten enthalten im wesentlichen 0,5 bis 30 Gew.-% einer oder mehrerer der erfindungsgemäßen Verbindungen, 0,5 bis 95 Gew.-% Wasser, 0,5 bis 95 Gew.-% eines organischen Lösungsmittels oder Lösungsmittelgemisches, 0,5 bis 50 Gew.-% eines strahlungshärtbaren Bindemittels und gegebenenfalls 0 bis 10 Gew.-% eines Photoinitiators.

Hot-Melt-Tinten basieren meist auf Wachsen, Fettsäuren, Fettalkoholen oder Sulfonamiden, die bei Raumtemperatur fest sind und bei Erwärmen flüssig werden, wobei der bevorzugte Schmelzbereich zwischen ca. 60 und ca. 140°C liegt.

Hot-Melt-Ink-Jet-Tinten bestehen im Wesentlichen aus 20 bis 90 Gew.-% Wachs und 1 bis 10 Gew.-% einer oder mehrerer der erfindungsgemäßen Verbindungen. Weiterhin können 0 bis 20 Gew.-% eines zusätzlichen Polymers (als "Farbstofflöser"), 0 bis 5 Gew.-% Dispergiermittel, 0 bis 20 Gew.-% Viskositätsveränderer, 0 bis 20 Gew.-% Plastifizierer, 0 bis 10 Gew.-% Klebrigkeitszusatz, 0 bis 10 Gew.-% Transparenzstabilisator (verhindert z.B. die Kristallisation des Wachses) sowie 0 bis 2 Gew.-% Antioxidans enthalten sein.

Die erfindungsgemäßen Drucktinten, insbesondere Ink-Jet-Tinten, können hergestellt werden, indem die erfindungsgemäßen Verbindungen in das Mikroemulsionsmedium, in das nicht-wässrige Medium oder in das Medium zur Herstellung der UV-härtbaren Tinte oder in das Wachs zur Herstellung einer Hot-Melt-Ink-Jet-Tinte eindispergiert werden.
Zweckmäßigerweise werden die dabei erhaltenen Drucktinten für Ink-Jet-Anwendungen anschließend filtriert (z.B. über einen 1 µm Filter).

Weiterhin sind die erfindungsgemäßen Verbindungen auch als Farbmittel für Farbfilter, sowohl für die additive wie auch für die subtraktive Farberzeugung, sowie als Farbmittel für elektronische Tinten ("electronic inks" bzw. "e-inks") oder "electronic paper" ("e-paper") geeignet.
Bei der Herstellung so genannter Farbfilter, sowohl reflektierender wie durchsichtiger Farbfilter, werden Pigmente in Form einer Paste oder als pigmentierte Photoresists in geeigneten Bindemitteln (Acrylate, Acrylester, Polyimide, Polyvinylalkohole, Epoxide, Polyester, Melamine, Gelatine, Caseine) auf die jeweiligen LCD-Bauteilen (z.B. TFT-LCD= Thin Film Transistor Liquid Crystal Displays oder z.B. ((S) TN-LCD = (Super) Twisted Nematic-LCD) aufgebracht. Neben einer hohen Thermostabilität ist für eine stabile Paste bzw. einem pigmentierten Photoresist auch eine hohe Pigmentreinheit Voraussetzung. Darüber hinaus können die pigmentierten Color Filter auch durch Ink Jet-Druckverfahren oder andere geeignete Druckverfahren aufgebracht werden.

Zur Beurteilung der Eigenschaften der nach der Erfindung hergestellten Pigmente auf dem Lacksektor wurde aus der Vielzahl der bekannten Lacke ein aromatenhaltiger Alkydmelaminharzlack (AM-Lack) auf Basis eines mittelöligen, nicht trocknenden Alkydharzes ausgewählt.

Die erfindungsgemäßen Pigmente zeichnen sich durch gute Echtheitseigenschaften aus, insbesondere besitzen sie gleichzeitig hohe Farbstärken und hohe Lösemittelechtheiten oder hohe Lichtechtheiten. Sie enthalten keine für die Umwelt bedenklichen Schwermetalle. Die aufgezählten Eigenschaften qualifizieren die erfindungsgemäßen Pigmente insbesondere zum Einsatz als Farbmittel im Druckbereich (insbesondere Druckfarben, Herstellung von Ink-Jet Tinten) sowie zur Verwendung in Lacken und in Kunststoffen, Farbfiltern und Tonern.

In den nachfolgenden Beispielen sind mit dem Begriff Equivalente Molequivalente gemeint.

### Beispiele:

### A) Herstellung von 1,4-Phenylendimalonsäure:

Zu einer auf 0 bis 5°C gekühlten Suspension aus 78,9 g (0,20 mol) 1,4-Phenylendimalonsäuretetraethylester in 800 ml Eiswasser werden 56,8 g (0,86 mol) KOH (Pulver, 85 %ig) gelöst in 800 ml Wasser innerhalb von 20 min tropfenweise zugegeben. Anschließend wird für 2,5 h unter Rückfluss erwärmt wobei ca. 180 ml des Lösungsmittels aus dem Reaktionsgemisch abdestilliert werden. Nach dem Abkühlen wird das Reaktionsgemisch im Eisbad gekühlt und tropfenweise mit 1000 ml 2N HCl versetzt (pH 1) und anschließend mit 8 x 500 ml Ethylacetat extrahiert. Die vereinigten Ethylacetat-Extrakte werden über Na₂SO₄ getrocknet, und i. Vak. zur Trockene eingeengt. Umkristallisieren aus Tetrahydrofuran/Cyclohexan liefert 41,2 g (73 %), farblose Kristalle vom Schmp. 251- 254°C.
Das erhaltene Produkt wird für die Herstellung des 1,4-Phenylendimalonylchlorids eingesetzt.

### B) Allgemeine Vorschrift zur Herstellung der Malonylchlorid-Derivate aus Methylmalonsäure, Phenylmalonsäure und 1,4-Phenylendimalonsäure:

Zu einer Lösung aus 1 Equivalent des entsprechenden Malonsäurederivates in Dichlormethan werden 4,5 Equivalente Phosphorpentachlorid gegeben und für 2 h bei etwa 20°C gerührt. Gelöstes HCl-Gas wird i. Vak. (ca. 200 mbar, 45 min) entfernt. Anschließend wird das gesamte Reaktionsgemisch bei etwa 40°C i. Vak. zur Trockene eingeengt. Das erhaltene Malonylchlorid wird direkt in die Synthese der mesoionischen Verbindungen ("Variante A-D") eingesetzt.

### C) Allgemeine Vorschrift zur Herstellung mesoionischer Pigmente

Die jeweiligen Reaktionsbedingungen sind in Tabelle 1 und Tabelle 2 zusammengefasst.

### Variante A

Zu 2,5 Equivalenten Malonylchlorid-Derivat (Herstellung siehe Allgemeine Vorschrift), gelöst in dem verwendeten Lösungsmittel, wird bei Raumtemperatur eine Lösung aus 1 Equivalent Amidin in dem verwendeten Lösungsmittel im Verlauf von 1,5-3,0 h langsam zugegeben. Nach beendeter Zugabe wird das Reaktionsgemisch für 18-20 h bei Raumtemperatur gerührt. Nach dem Abkühlen wird filtriert und mit dem verwendeten Lösungsmittel gewaschen und getrocknet (80-100°C).

### Variante B

Zu 4 Equivalenten Malonylchlorid-Derivat (Herstellung siehe Allgemeine Vorschrift), gelöst in dem verwendeten Lösungsmittel, wird bei 50-60°C eine Lösung/Suspension aus 1 Equivalent Amidin und 4,5 Equivalenten Triethylamin in dem verwendeten Lösungsmittel im Verlauf von 0,5-3,0 h langsam zugegeben. Nach beendeter Zugabe wird das Reaktionsgemisch für 18-20 h unter Rückfluss gerührt. Nach dem Abkühlen wird filtriert und mit dem verwendeten Lösungsmittel gewaschen. Der erhaltene Filterpresskuchen wird in Wasser gerührt, filtriert, mit Wasser salzfrei gewaschen und getrocknet (80-100°C).

### Variante C

Zu 4,0 Equivalenten Malonylchlorid-Derivat (Herstellung siehe Allgemeine Vorschrift), gelöst in dem verwendeten Lösungsmittel, wird bei 20°C unter starkem Rühren 1 Equivalent Amidin zugegeben. Anschließend wird eine Mischung aus 4,5 Equivalenten Triethylamin in dem verwendeten Lösungsmittel im Verlauf von 0,5-3,0 h langsam zugegeben. Nach beendeter Zugabe wird das Reaktionsgemisch für 18-20 h unter Rückfluss gerührt. Nach dem Abkühlen wird filtriert und mit dem verwendeten Lösungsmittel gewaschen. Der erhaltene Filterpresskuchen wird in Wasser gerührt, filtriert, mit Wasser salzfrei gewaschen und getrocknet (80-100°C).

### Variante D

Unter Rückfluss wird zu einer Lösung aus 2,2 Equivalenten Amidin und 4,5 Equivalenten Triethylamin in dem angegebenen Lösungsmittel, tropfenweise im Verlauf von 0,5-2,5 h eine Lösung aus 1 Equivalent 1,4-Phenylendimalonylchlorid (Herstellung siehe Allgemeine Vorschrift) im angegebenen Lösungsmittel zugegeben. Das Reaktionsgemisch wird unter Rückfluss für 18-20 h gerührt. Nach dem Abkühlen wird filtriert und mit dem verwendeten Lösungsmittel gewaschen. Der erhaltene Filterpresskuchen wird in Wasser gerührt, filtriert und mit Wasser salzfrei gewaschen und getrocknet (80-100°C).

### D) Nachbehandlung der Pigmente

Zur Nachbehandlung wird das erhaltene Rohpigment ("Variante A-D") in dem verwendeten Lösungsmittel unter Rückflussbedingungen gerührt, filtriert, gewaschen, getrocknet (80-100°C) und gemahlen.

### Beispiele 1 bis 14:

### Beispiele 1 und 2 sind Vergleichsbeispiele.

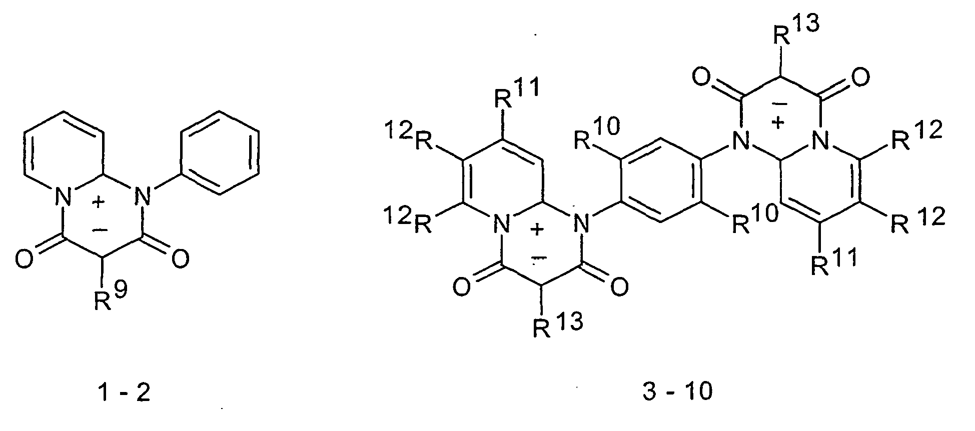

| Beispiel | R⁹ | R¹⁰ | R¹¹ | R¹² | R¹³ |
|---|---|---|---|---|---|
| 1 | Me | -- | -- | -- | -- |
| 2 | Ph | -- | -- | -- | -- |
| 3 | -- | H | H | H | Me |
| 4 | -- | H | H | H | Ph |
| 5 | -- | Me | H | H | Me |
| 6 | -- | Me | H | H | Ph |
| 7 | -- | Cl | H | H | Me |
| 8 | -- | Cl | H | H | Ph |
| 9 | -- | H | Me | H | Ph |
| 10 | -- | H | H | 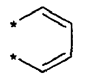 | Ph |

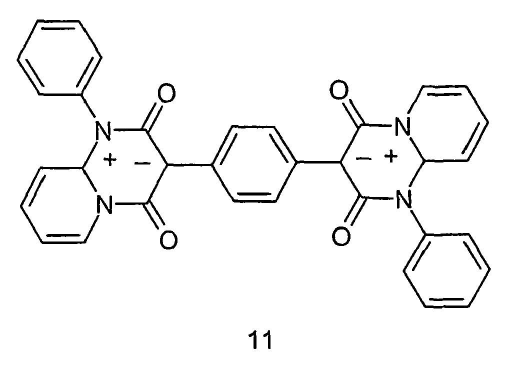

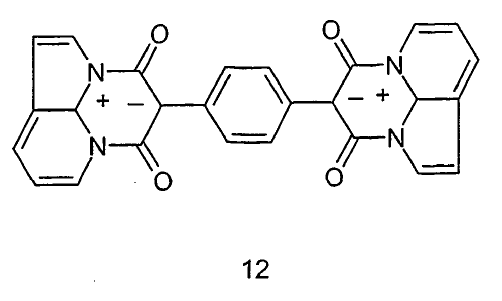

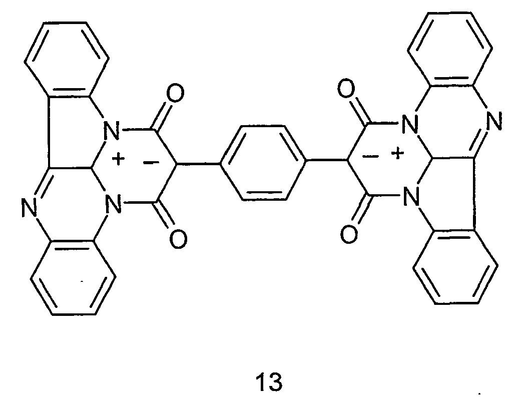

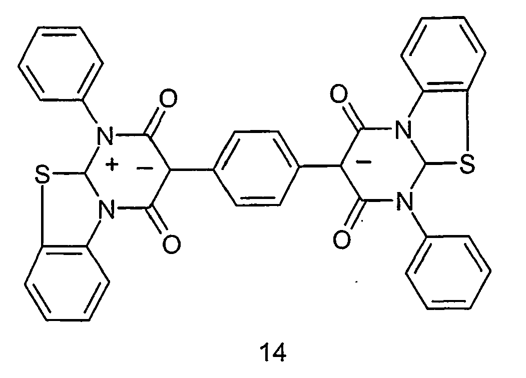

**Tabelle 1 - Reaktionsbedingungen / Nachbehandlung / Ausbeuten**

| Bsp. | Variante | Malonylchlorid | Ansatzgröße [mol] ^{[1]} | Lösungsmittel (Malonylchlorid/ Amidin) | Nachbehandlung ^{[2]} | Ausb. [%] |
|---|---|---|---|---|---|---|
| 1 | A | Methyl- | 0.2 | CH₂Cl₂ (300/250 ml) | -- | 53 |
| 2 | A | Phenyl- | 0.2 | CH₂Cl₂ (300/150 ml) | -- | 34 |
| 3 | B | Methyl- | 0.125 | MEK (400/1850 ml) | DMF/MeOH | 71 |
| 4 | B | Phenyl- | 0.1 | MEK (300/1500 ml) | CHCl₃ | 89 |
| 5 | C | Methyl- | 0.1 1 | MEK (500/200 ml) | DMF/CHCl₃ | 32 |
| 6 | C | Phenyl- | 0.1 | MEK (500/200 ml) | DMF | 89 |
| 7 | B | Methyl- | 0.074 | CHCl₃ (250/1000 ml) | DMF/j-BuOH | 38 |
| 8 | B | Phenyl- | 0.1 | CHCl₃ (250/1500 ml) | DMF | 66 |
| 9 | B | Phenyl- | 0.08 | CHCl₃ (240/1500 ml) | DMF | 55 |
| 10 | C | Phenyl- | 0.095 | CHCl₃ (500/200 ml) | DMF | 87 |
| 11 | D | 1,4-Phenylendi- | 0.022 | CH₂Cl₂ (50/75 ml) | DMF | 64 |
| 12 | D | 1,4-Phenylendi- | 0.022 | CH₂Cl₂ (50/75 ml) | -- | 78 |
| 13 | D | 1,4-Phenylendi- | 0.022 | THF (50/75 ml) | -- | 89 |
| 14 | D | 1,4-Phenylendi- | 0.022 | CH₂Cl₂ (50/75 ml) | DMSO | 27 |

| | | | | | | |
|---|---|---|---|---|---|---|
| [1] bezogen auf das Amidin; [2] unter Rückfluss 30-60 min. DMF = Dimethylformamid, DMSO = Dimethylsulfoxid, MEK = Methylethylketon, MeOH = Methanol, THF = Tetrahydrofuran. | | | | | | |

**Tabelle 2 - Physikalische Daten / Pigmenteigenschaften**

| Bsp. | Pigmentfarbe | Festkörperfluoreszenz^{[3]} | Schmp. [°C] | MALDI-TOF-MS (DHB, m/z, pos. Modus) |
|---|---|---|---|---|
| 1 | grünstichig Gelb | ja | 275-280 | 253.2 [M+H], 225.2 [M+H-CO] |
| 2 | grünstichig Gelb | ja | 237-241 | 314.4 [M], 286.4 [M-CO] |
| 3 | grünstichig Gelb | ja | 374-379 | 427.4 [M], 399.4 [M-CO] |
| 4 | Gelb | ja | 352-357 | 551.6 [M], 522.6 [M-CO] |
| 5 | Gelb | ja | 377-381 | 455.6 [M+H] |
| 6 | Gelb | ja | 368-375 | 479.8 [M+H], 550.8 [M-CO] |
| 7 | Gelb | ja | 377-381 | 493.1, 495.2, 497.1 [M-H], 461.2, 459.2 [M-HCl] |
| 8 | Gelb | ja | 389-393 | 618.1, 620.1, 622.1 [M], 619.1, 621.1, 623.1, [M+H], 590.1, 592.1 [M-CO] |
| 9 | Gelb | ja | 386-391 | 579.2 [M+H], 550.2 [M-CO] |
| 10 | Orange | ja | 312-315 | 651.8 [M+H], 622.7 [M-CO] |
| 11 | rotstichig Gelb | ja | 372-375 | 550.3 [M], 522.3 [M-CO] |
| 12 | Rot | nein | > 400 | 446.3 [M] |
| 13 | dunkel Grün | nein | > 400 | 648.8 [M], 621.8 [M+H-CO] |
| 14 | Gelb | nein | 359-364 | 662.5 [M], 634.5 [M-CO] |

| | | | | |
|---|---|---|---|---|
| ^{[3]} im UV-Licht bei 366 nm. DHB = 2,5-Dihydroxybenzoesäure MALDI-TOF-MS = Matrix Assisted Laser Desorption Ionisation - Time of Flight - Mass Spectrometry. | | | | |

### Anwendungsbeispiele:

In Tabelle 3 sind die Lösemittelechtheiten und Farbstärken der mesoionischen Vergleichsverbindungen 1 und 2 im Vergleich mit den Eigenschaften der durch Dimerisierung erhaltenen erfindungsgemäßen Verbindungen 4 und 11 angegeben.

**Tabelle 3**

| Bsp. | Pigmentfarbe | Lösemittelechtheit | Farbstärke |
|---|---|---|---|
| 1 | grünstichig Gelb | 3 | 0,10 |
| 2 | grünstichig Gelb | 2 | 0,06 |
| 4 | Gelb | 3-4 | 2,40 |
| 11 | rotstichig Gelb | 4-5 | 1,58 |

Die Lösemittelechtheit wurde gegen die 5-stufige Grauskala nach DIN 54002 ermittelt.
Die Farbstärke gibt an, wie viele Teile TiO₂ notwendig sind, um 1 Teil Buntpigment auf 1/3 Standard-Farbtiefe zu bringen: 1 : x TiO₂ (Farbstärke und ihre Messung ist definiert nach DIN EN ISO 787-26).

## Patentansprüche

1. Dimere Verbindung der Formel (11) 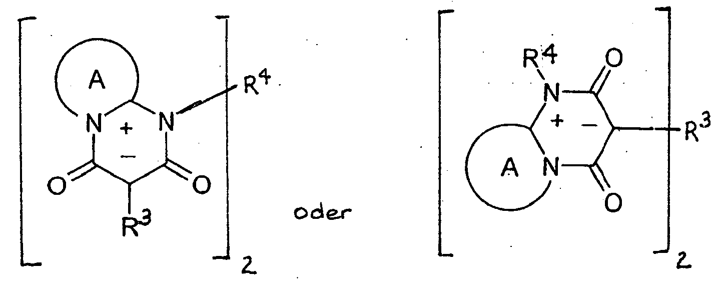 wobei
der Ring A ein fünf- oder sechsgliedriger heteroaromatischer Ring der Struktur A1 bis A7 ist 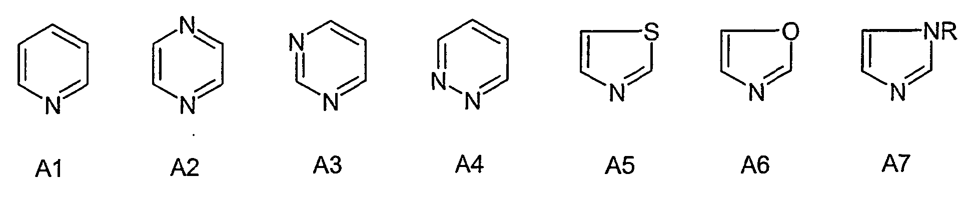 wobei die Ringe A1 bis A7 unsubstituiert, mit C₁-C₄-Alkyl oder Phenyl substituiert und/oder mit einem Benzolring anelliert sind,
einer der Reste R³ oder R⁴ ein unsubstituiertes oder ein mit Alkyl, Alkoxy und/oder Halogen substituiertes Phenylenradikal,
der andere der Reste R³ oder R⁴ C₁-C₄-Alkyl, C₅-C₆-Cycloalkyl, ein unsubstituiertes oder ein mit Alkyl, Alkoxy, Nitro, Phenyl, Alkoxycarbonyl, Dialkylamino, Dialkylaminocarbonyl, Alkylaminocarbonyl, Aminocarbonyl und/oder Halogen substituiertes Phenyl, Benzyl, Benzanilid, C₅-C₆-Cycloalkyl oder Naphthyl; oder wobei die Gruppe NR⁴ zusammen mit dem Ring A einen 5- oder 6-gliedrigen Heterocyclus, der noch mit einem Benzolring anelliert sein kann, bildet, und R³ ein unsubstituiertes oder ein mit Alkyl, Alkoxy und/oder Halogen substituiertes Phenylenradikal ist; und R ein C₁-C₄-Alkyl oder Phenyl bedeutet.

2. Verbindung nach Anspruch 1, **gekennzeichnet durch** die allgemeinen Formeln (IIa) und (IIb) 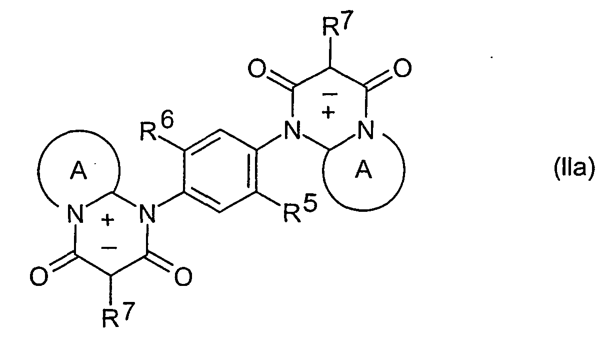 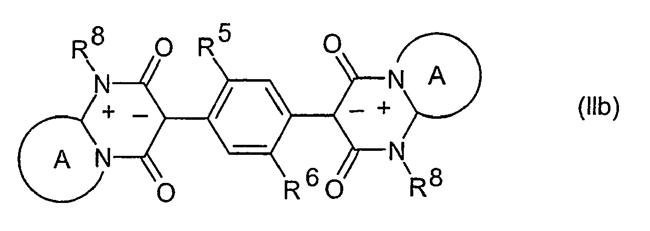 wobei
R⁵ und R⁶ unabhängig voneinander, für Wasserstoff, C₁-C₄-Alkyl, C₁-C₄-Alkoxy oder Halogen stehen;
R⁷ und R⁸ für C₁-C₄-Alkyl, C₅-C₆-Cycloalkyl, ein unsubstituiertes oder ein mit 1, 2, 3 oder 4 Resten aus der Gruppe C₁-C₄-Alkyl, C₁-C₄-Alkoxy, Nitro, Phenyl, C₁-C₄-Alkoxycarbonyl, Di(C₁-C₃-Alkyl)amino, Di(C₁-C₃-Alkyl)-aminocarbonyl, (C₁-C₃-Alkyl)-aminocarbonyl, Aminocarbonyl und/oder Chlor substituiertes Phenyl, Benzyl, Benzanilid oder Naphthyl stehen;
oder wobei die Gruppe NR⁸ zusammen mit dem Ring A einen 5- oder 6-gliedrigen Heterocyclus, der noch mit einem Benzolring anelliert sein kann, bildet.

3. Verbindung nach Anspruch 2, **dadurch gekennzeichnet, dass** R⁵ und R⁶ gleich oder verschieden sind und Wasserstoff, Methyl oder Chlor bedeuten.

4. Verbindung nach einem oder mehreren der Ansprüche 1 bis 3, **dadurch gekennzeichnet, dass**
R³, R⁴, R⁷ und R⁸ ein substituiertes Phenylradikal aus der Gruppe 1-, 2-, 3-Methyl-, Ethyl-, Methoxy-, Ethoxy-, Diethylamino-, Chlor-, 2,5-Dichlor-, 3-Chlor-4-methyl-, 3-Chlor-4-methoxy- und 4-Nitrophenyl ist.

5. Verbindung nach mindestens einem der Ansprüche 1 bis 4, **gekennzeichnet durch** die Formel (V) 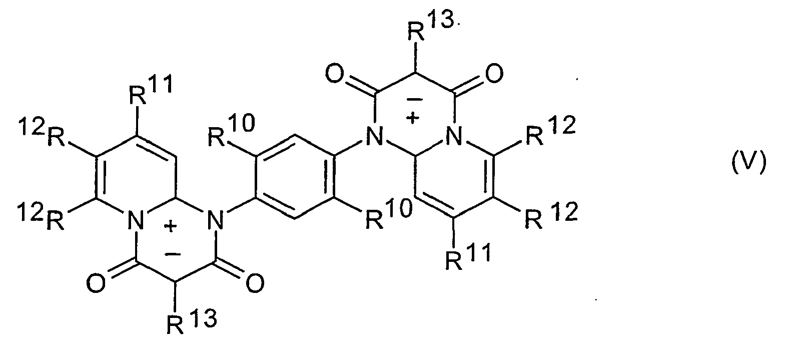 worin
R¹⁰ Wasserstoff, Methyl oder Chlor,
R¹¹ Wasserstoff oder Methyl,
R¹² Wasserstoff oder zwei benachbarte Reste R¹² gemeinsam den zweiwertigen Rest C₄H₄, und
R¹³ Methyl oder Phenyl bedeuten.

6. Verbindung nach Anspruch 1 oder 2, **gekennzeichnet durch** die Formel (11), (12), (13), oder (14)

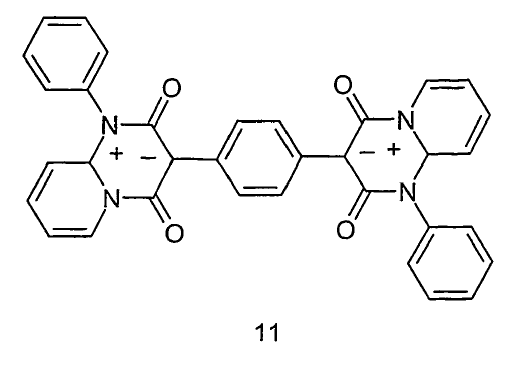

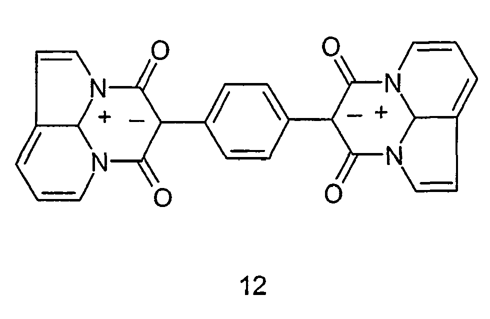

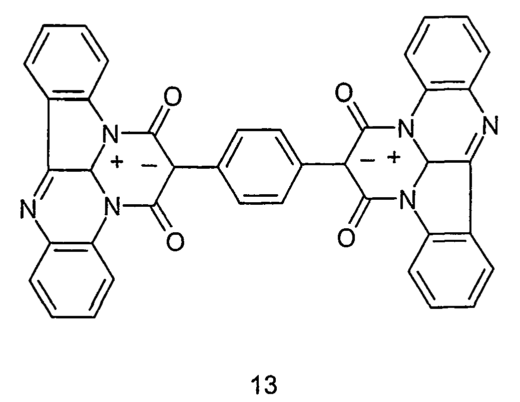

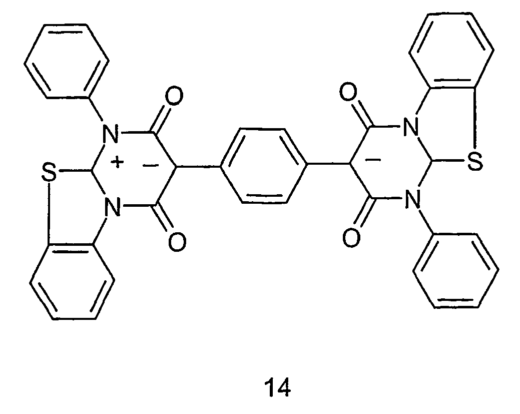

7. Verfahren zur Herstellung einer Verbindung nach einem oder mehreren der Ansprüche 1 bis 6, **dadurch gekennzeichnet, dass** man entweder
(a) ein Equivalent der Verbindung der Formel (III) mit n gleich 2 mit etwa zwei Equivalenten der Verbindung der Formel (IV) mit m gleich 1; oder
(b) ein Equivalent der Verbindung der Formel (IV) mit m gleich 2 mit etwa zwei Equivalenten der Verbindung der Formel (III) mit n gleich 1, kondensiert

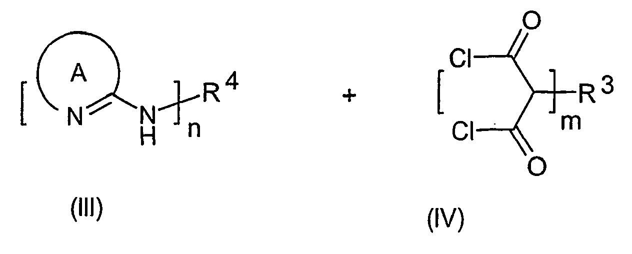

8. Verfahren nach Anspruch 7, **dadurch gekennzeichnet, dass** die Kondensation in Gegenwart einer Base erfolgt.

9. Verfahren nach Anspruch 7 oder 8, **dadurch gekennzeichnet, dass** die Verbindung der Formel (II) einer Feinverteilung und/oder Lösemittelbehandlung unterzogen wird.

10. Verwendung einer Verbindung nach einem oder mehreren der Ansprüche 1 bis 6 zum Pigmentieren von hochmolekularen organischen Materialien natürlicher oder synthetischer Herkunft.

11. Verwendung nach Anspruch 10 zum Pigmentieren von Kunststoffen, Harzen, Lacken, Anstrichfarben, elektrophotographischen Tonern und Entwicklern, Elektretmaterialien, Farbfiltern, Tinten, Ink-Jet-Tinten, Druckfarben und Saatgut.

## Claims

1. A dimeric compound of formula (II) 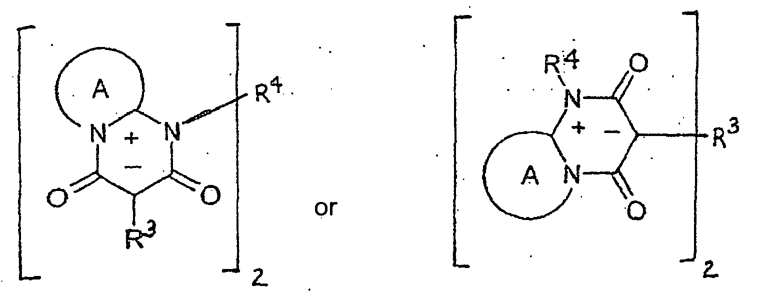 where the ring A is a five- or six-membered heteroaromatic ring of structure A1 to A7 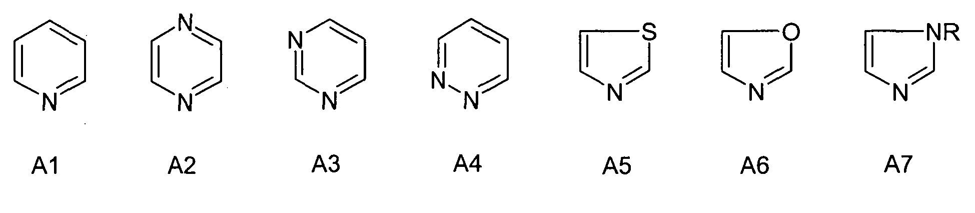 where the rings A1 to A7 are unsubstituted, C₁-C₄-alkyl or phenyl substituted and/or fused with a benzene ring,
one of R³ and R⁴ is an unsubstituted or alkyl-, alkoxy- and/or halogen-substituted phenylene radical,
the other one of R³ and R⁴ is C₁-C₄-alkyl, C₅-C₆-cycloalkyl, an unsubstituted or alkyl-, alkoxy-, nitro-, phenyl-, alkoxycarbonyl-, dialkylamino-, dialkylaminocarbonyl-, alkylaminocarbonyl-, aminocarbonyl- and/or halogen-substituted phenyl, benzyl, benzanilide, C₅-C₆-cycloalkyl or naphthyl; or where the NR⁴ group may combine with the A ring to form a 5- or 6-membered heterocycle which may be additionally fused with a benzene ring, and R³ is an unsubstituted or alkyl-, alkoxy- and/or halogen-substituted phenylene radical; and R is C₁-C₄-alkyl or phenyl.

2. A compound according to claim 1, **characterized by** the general formulae (IIa) and (IIb) 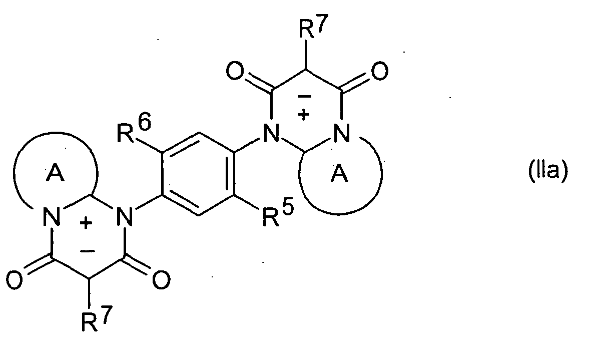 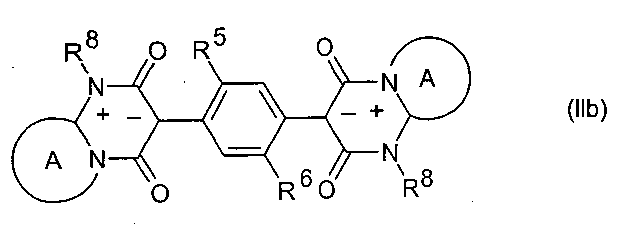 where
R⁵ and R⁶ are independently hydrogen, C₁-C₄-alkyl, C₁-C₄-alkoxy or halogen;
R⁷ and R⁸ are C₁-C₄-alkyl, C₅-C₆-cycloalkyl, a phenyl, benzyl, benzanilide or naphthyl that is unsubstituted or substituted by 1, 2, 3 or 4 radicals selected from the group consisting of C₁-C₄-alkyl, C₁-C₄-alkoxy, nitro, phenyl, C₁-C₄-alkoxycarbonyl, di(C₁-C₃-alkyl)amino, di(C₁-C₃-alkyl)aminocarbonyl, (C₁-C₃-alkyl)aminocarbonyl, aminocarbonyl and/or chlorine;
or where the NR⁸ group combines with the A ring to form a 5- or 6-membered heterocycle which may be additionally fused with a benzene ring.

3. A compound according to claim 2, wherein R⁵ and R⁶ are the same or different and are each hydrogen, methyl or chlorine.

4. A compound according to one or more of claims 1 to 3, wherein R³, R⁴, R⁷ and R⁸ is a substituted phenyl radical from the group consisting of 1-, 2-, 3-methyl-, ethyl-, methoxy-, ethoxy-, diethylamino-, chloro-, 2,5-dichloro-, 3-chloro-4-methyl-, 3-chloro-4-methoxy- and 4-nitrophenyl.

5. A compound according to at least one of claims 1 to 4, **characterized by** formula (V) 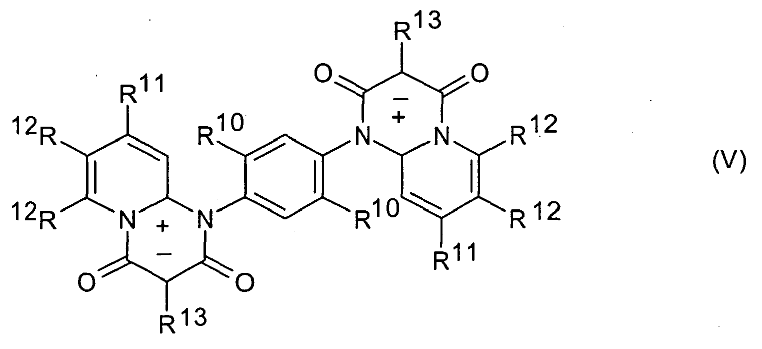 where
R¹⁰ is hydrogen, methyl or chlorine,
R¹¹ is hydrogen or methyl,
R¹² is hydrogen, or two adjacent R¹² radicals together are a divalent C₄H₄ radical, and
R¹³ is methyl or phenyl.

6. A compound according to claim 1 or 2, **characterized by** the formula (11), (12), (13) or (14)

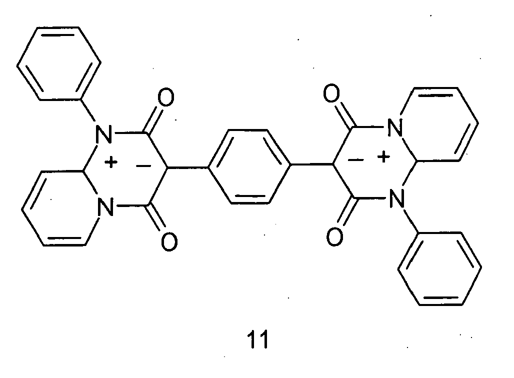

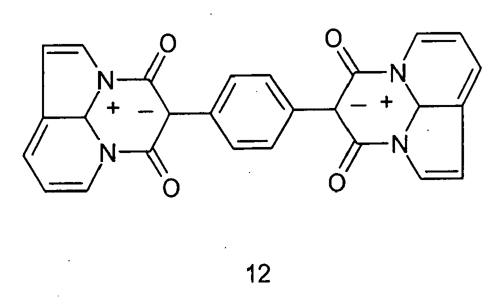

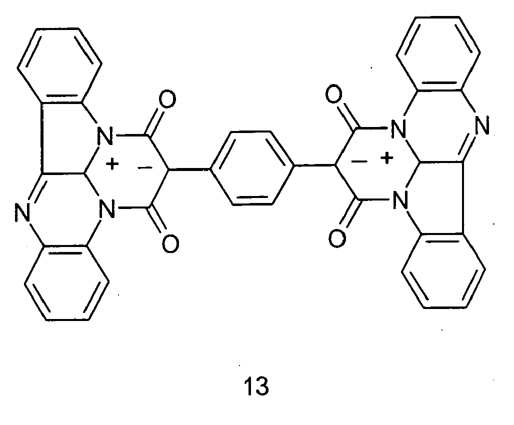

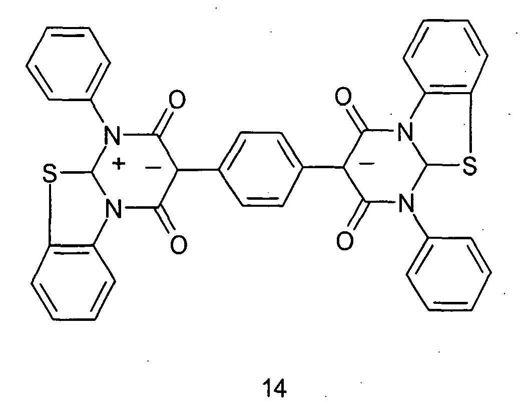

7. A process for preparing a compound according to one or more of claims 1 to 6, which comprises condensing either
(a) one equivalent of the compound of formula (III) where n is 2 with about two equivalents of the compound of formula (IV) where m is 1; or
(b) one equivalent of the compound of formula (IV) where m is 2 with about two equivalents of the compound of formula (III) where n is 1,

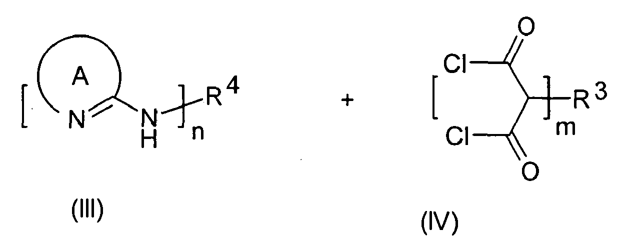

8. The process according to claim 7 wherein the condensing is effected in the presence of a base.

9. The process according to claim 7 or 8 wherein the compound of formula (II) is subjected to a fine-dividing operation and/or solvent treatment.

10. The use of a compound according to one or more of claims 1 to 6 for pigmenting macromolecular organic materials of natural or synthetic origin.

11. The use according to claim 10 for pigmenting plastics, resins, coatings, paints, electrophotographic toners and developers, electret materials, color filters, liquid inks, inkjet inks, printing inks and seed.

## Revendications

1. Composé dimère de formule (II) 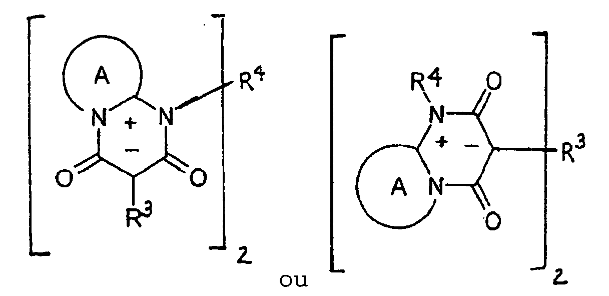 dans lequel le cycle A est un cycle hétéroaromatique à cinq ou six éléments de structure A1 à A7 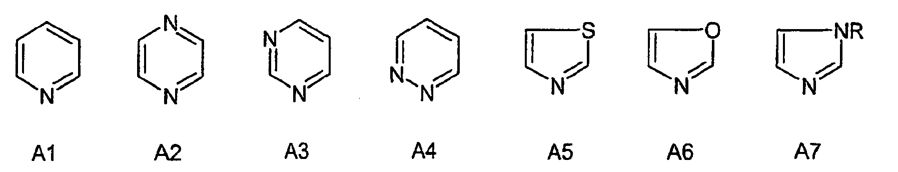 dans lesquelles
les cycles A1 à A7 sont non substitués, substitués par un groupe alkyle en C₁ à C₄ ou phényle et/ou condensés avec un noyau benzénique,
l'un des groupes R³ ou R⁴ représente un groupe phénylène non substitué ou substitué par un groupe alkyle, alcoxyle et/ou un atome d'halogène,
l'autre des groupes R³ ou R⁴ représente un groupe alkyle en C₁ à C₄, cycloalkyle en C₅ à C₆, un groupe phényle, benzyle, benzanilide, cycloalkyle en C₅ à C₆ ou naphtyle non substitué ou substitué par un groupe alkyle, alcoxyle, nitro, phényle, alcoxycarbonyle, dialkylamino, dialkylaminocarbonyle, alkylaminocarbonyle, aminocarbonyle et/ou un atome d'halogène ;
ou dans lesquelles le groupe NR⁴, conjointement avec le cycle A, forme un hétérocycle à cinq ou six éléments, qui peut en plus être condensé avec un noyau benzénique, et R³ représente un groupe phénylène non substitué ou substitué par un groupe alkyle, alcoxyle et/ou un atome d'halogène ; et R représente un groupe alkyle en C₁ à C₄ ou phényle.

2. Composé selon la revendication 1, **caractérisé par** les formules générales (IIa) et (IIb) 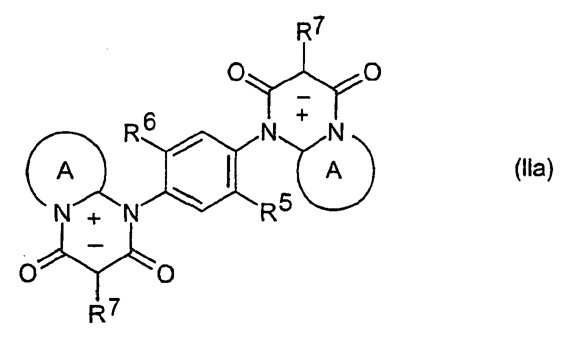 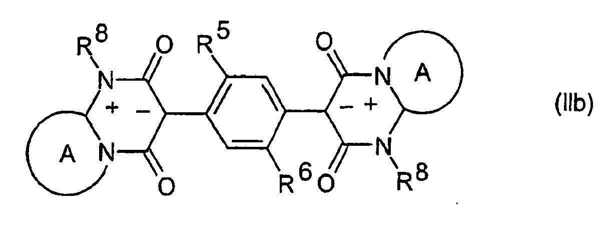 dans lesquelles
R⁵ et R⁶ représentent, indépendamment l'un de l'autre, un atome d'hydrogène, un groupe alkyle en C₁ à C₄, alcoxyle en C₁ à C₄ ou un atome d'halogène ;
R⁷ et R⁸ représentent un groupe alkyle en C₁ à C₄, cycloalkyle en C₅ à C₆, un groupe phényle, benzyle, benzanilide ou naphtyle non substitué ou substitué par 1, 2, 3 ou 4 groupes choisis dans le groupe constitué par un groupe alkyle en C₁ à C₄, alcoxyle en C₁ à C₄, nitro, phényle, alcoxycarbonyle en C₁ à C₄, di(alkyle en C₁ à C₃)-amino, di(alkyle en C₁ à C₃)-aminocarbonyle, (alkyle en C₁ à C₃)-aminocarbonyle, aminocarbonyle et/ou un atome de chlore ;
ou dans lesquelles le groupe NR⁸, conjointement avec le cycle A, forme un hétérocycle à cinq ou six éléments, qui peut en plus être condensé avec un noyau benzénique.

3. Composé selon la revendication 2, **caractérisé en ce que** R⁵ et R⁶ sont identiques ou différents et représentent un atome d'hydrogène, un groupe méthyle ou un atome de chlore.

4. Composé selon l'une quelconque ou plusieurs des revendications 1 à 3, **caractérisé en ce que** R³, R⁴, R⁷ et R⁸ représentent un groupe phényle substitué choisi dans le groupe constitué par un groupe 1-, 2-, 3-méthyl-, éthyl-, méthoxy-, éthoxy-, diéthylamino-, chloro-, 2,5-dichloro-, 3-chloro-4-méthyl-, 3-chloro-4-méthoxy- et 4-nitro-phényle.

5. Composé selon au moins l'une quelconque des revendications 1 à 4, **caractérisé par** la formule (V) 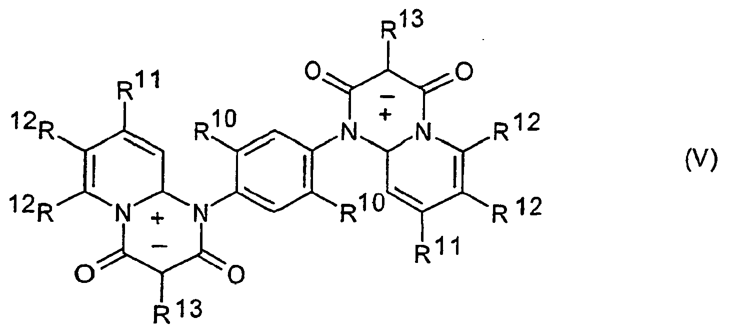 dans laquelle
R¹⁰ représente un atome d'hydrogène, un groupe méthyle ou un atome de chlore,
R¹¹ représente un atome d'hydrogène ou un groupe méthyle,
R¹² représente un atome d'hydrogène, ou deux groupes R¹² vicinaux forment ensemble le groupe bivalent C₄H₄, et
R¹³ représente un groupe méthyle ou phényle.

6. Composé selon la revendication 1 ou 2, **caractérisé par** la formule (11), (12), (13) ou (14)

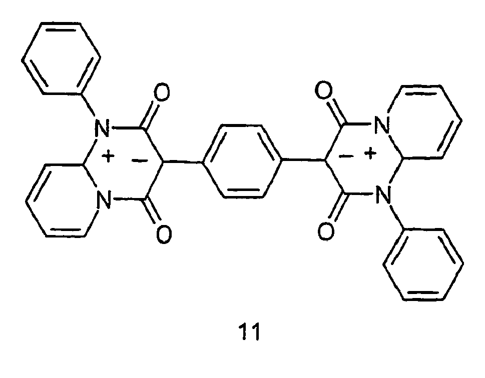

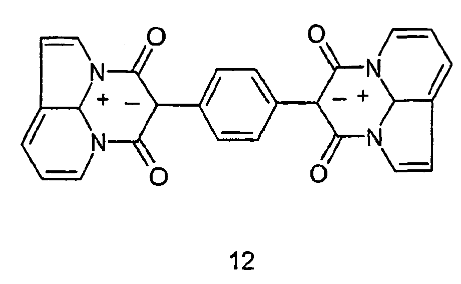

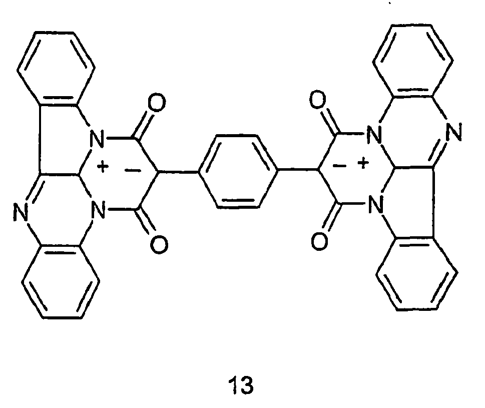

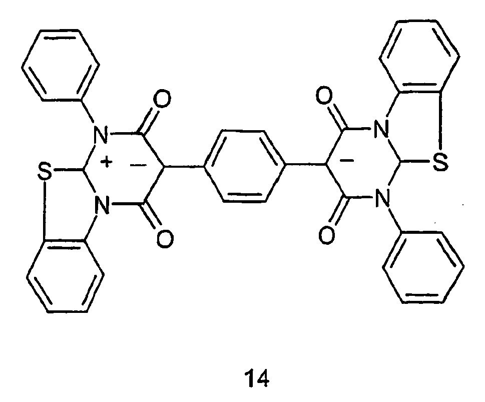

7. Procédé de préparation d'un composé selon l'une quelconque ou plusieurs des revendications 1 à 6, **caractérisé en ce que** l'on condense
(a) soit un équivalent de composé de formule (III), où n vaut 2, avec approximativement deux équivalents du composé de formule (IV), où m vaut 1 ;
(b) soit un équivalent du composé de formule (IV), où m vaut 2, avec approximativement deux équivalents du composé de formule (III), où n vaut 1

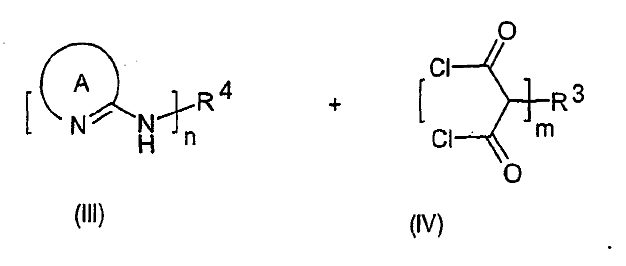

8. Procédé selon la revendication 7, **caractérisé en ce que** la condensation est réalisée en présence d'une base.

9. Procédé selon la revendication 7 ou 8, **caractérisé en ce que** le composé de formule (II) est soumis à une division fine et/ou à un traitement au solvant.

10. Utilisation d'un composé selon l'une quelconque ou plusieurs des revendications 1 à 6 pour pigmenter des matières organiques à haute masse moléculaire d'origine naturelle ou synthétique.

11. Utilisation selon la revendication 10 pour pigmenter des matières plastiques, des résines, des vernis, des peintures, des toners et des révélateurs électrophotographiques, des matériaux de type électret, des filtres colorés, des encres, des encres pour impression par jet d'encre, des encres d'imprimerie et des semences.
